(19) Europäisches Patentamt
European Patent Office
Office européen des brevets

(11) Publication number: **0 126 684 B1**

(12) **EUROPEAN PATENT SPECIFICATION**

(45) Date of publication of patent specification: **28.08.91**

(51) Int. Cl.5: **A61K 47/00**, A61K 9/06

(21) Application number: **84400982.9**

(22) Date of filing: **15.05.84**

# (54) Drug delivery system utilizing thermosetting gels.

(30) Priority: **16.05.83 US 495238**
**16.05.83 US 495239**
**16.05.83 US 495240**
**16.05.83 US 495321**

(43) Date of publication of application:
**28.11.84 Bulletin 84/48**

(45) Publication of the grant of the patent:
**28.08.91 Bulletin 91/35**

(84) Designated Contracting States:
**AT BE CH DE FR GB IT LI LU NL SE**

(56) References cited:
**AU-A- 482 821**
**CA-A- 1 072 413**
**FR-A- 1 598 998**

**N.F.ESTRIN et al.: "CTFA COSMETIC INGREDIENT DICTIONARY", Third Edition, The Cosmetic, Toiletry and Fragrance Association, Inc., Washington, D.C., US;**

(73) Proprietor: **MERCK & CO. INC.**
**126, East Lincoln Avenue P.O. Box 2000**
**Rahway New Jersey 07065-0900(US)**

(72) Inventor: **Haslam, John L.**
**RFD 2, Box 259-B**
**Lawrence Kansas 66044(US)**
Inventor: **Higuchi, Takeru**
**2811 Schwarz Road**
**Lawrence, Kansas 66044(US)**
Inventor: **Mlodozeniec, Arthur R.**
**1704 St. Andrews Drive**
**Lawrence Kansas 66044(US)**

(74) Representative: **Ahner, Francis et al**
**CABINET REGIMBEAU, 26, avenue Kléber**
**F-75116 Paris(FR)**

EP 0 126 684 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

A goal of pharmaceutics is to efficiently deliver a therapeutic drug to a patient by administering it to a body cavity, by, topical, injectable or ophthalmic administration to the site of action. Over the years, numerous methods have been developed in an attempt to either develop a new method or improve the conventional method in achieving this goal.

A major loss of drugs administered to the eye is via the lacrimal drainage system so that only a small fraction of the dose remains in the eye for an extended period of time from a liquid drop formulation. Different approaches have been taken to slow down this rapid loss of drug by using viscous solutions, gels, ointments and solid inserts.

Improvement in drug delivery has been achieved by these methods especially with the use of solid inserts where a large reduction in dose is possible while achieving the same therapeutic response as a liquid drop which must be administered more frequently and at higher drug concentration.

A principal advantage of the ophthalmic mode of administration is that it permits the accurate, reproducible unit dosing of a drug or active entity by using volumetric fluid delivery of the dosage prescribed while effecting the ultimate delivery of a semi-solid or rigid-gel state. Conventionally, it is not possible to deliver preformed gels from multiple dose containers readily by volumetric means. Gravimetric dosing is thus required to achieve uniform content in delivering reproducible quantities. Conventionally, voids and packing or consolidation problems result when administering semi-solid preparations volumetrically. The present ophthalmic mode of administration provides extremely accurate and uniform content of dose which is critical for many potent drugs.

A significant disadvantage to a solid insert however is that many patients have a difficult time inserting a solid object into the cul-de-sac of the eye and removing said solid object.

We describe here a unique drug delivery system which at room temperature and lower has the property of a liquid but when administered topically to the skin becomes a semi solid or gel when warmed by the body. The advantages of such a system for application to the skin are (1) the drug delivery system can be applied to tender, sensitive skin by a pour on application with no need to rub the area to obtain coverage, and (2) a liquid will flow into crevices and pores providing intimate contact with the skin.

When gelled, the drug delivery system remains in place with a flexible covering to the area of skin on which it is applied. Drugs are delivered to the skin from the gel but these drugs can also be removed by running cool water over the area. Such a drug delivery system will have application especially for burn patients where application and removal of drugs require gentle techniques because of the tender surface to which application is to be made.

Another approach to these problems is to use a formulation which is a liquid at room temperature but which forms a semi-solid when warmed to body temperatures. Such a system has been described in U.S. Patent No. 4,138,373 using "Pluronic® polyols" as the thermally gelling polymer. In this system the concentration of polymer is adjusted to give the desired sol-gel transition temperature, that is lower concentration of polymer gives a higher solution-gel (sol-gel) transition temperature. However with the currently commercially available "Pluronic®" polymers the ability to obtain a gel of the desired rigidity is limited while maintaining the desired sol-gel transition temperature at physiologically useful temperature ranges near 26-35 °C.

Similarly Canadian patent 1072413 which relates to (poloxamer) gel systems with gelling temperatures higher than room temperature uses additives to bring about the gelling characteristics of the polymer which contains therapeutic or other type agents. Also in this Canadian patent "Tetronic®" polymers are used as additive agents rather than the primary polymeric agent as in the instant case.

The state of the art can also be further illustrated by FR-A-1 598 998 which discloses gel compositions, the liquid gel transition temperature of which being not controllable.

### SUMMARY OF THE INVENTION

The present invention provides a pharmaceutical vehicle which is useful in delivering pharmacologically active medicaments to a body orifice such as the rectum, vagina, urethra, external auditory meatus, nasal passage or buccal or oral cavity; by injecting into the patient's body (human and animal) via subcutaneous or intramuscular injection; to the skin of a patient (human and animal) for topical, dermal and/or transdermal delivery of candidate drugs; and to the eye and in some cases as in dry eye using the vehicle alone. The drug delivery system consists of a clear physiologically-acceptable liquid which forms a semi-solid "gel" at

human body temperatures. The sol-gel transition temperature and rigidity of the gel can be modified by changes in polymer concentration combined with the pH and ionic strength of the solution.

We describe herein a unique drug delivery system which at room temperature has the properties of a liquid, but when administered by injection (subcutaneously or intramuscularly), topically, rectally, nasally, vaginally or optically or to the oral cavity, buccal pouch or urethral lumen, changes to a semi-solid or gel when warmed by the body. The advantages of such a system are: the convenience of handling a liquid during the administration phase including the property of a liquid to make intimate contact before gel formation and after gelling prolonging the release time of the drug at the site of administration thereby resulting in a reduced therapeutic effective amount of said drug being administered. The gelling characteristics of this system also enhance the retention of product formulations within body cavities or lumens which might normally be rapidly depleted if dosed exclusively as liquids. The injection formulation when administered as a liquid will flow within soft tissue and after gelling will respond as a deformable gel so as to minimize much of the discomfort associated with solids injection and encapsulation. The gelled injection site may also serve as a drug depot location. The dose sparing ability of such a drug delivery system is realized when serious side effects of some drugs are reduced.

The drug delivery system consists of a clear physiologically acceptable liquid which forms a semi-solid or gel at body temperature. The unique gelling characteristics of the various formulations permit retention of the system at skin interfaces which might otherwise be rapidly depleted of liquid drug delivery systems in areas such as between fingers or toes, in groin areas, under breasts or arms or on the scalp. The sol-gel transition temperature and rigidity of the gel can be modified by changes in polymer concentration combined with the pH and ionic strength of the solution.

It has been discovered that certain polymers are useful vehicles having the properties set forth above. The polymers are tetra substituted derivatives of ethylene diamine (poloxamine, w = 2 in Formula I), propylene diamine (w = 3), butylene diamine (w = 4), pentylene diamine (w = 5) or hexylene diamine (w = 6). The substituents are block copolymers of poly(oxypropylene) and poly(oxyethylene) of various chain lengths and ratios x to y in the general formula of the polymer shown below.

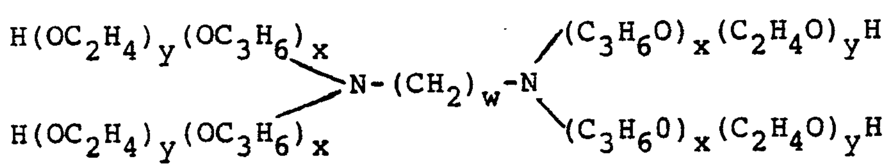

Formula I

wherein w is an integer from 2 through 6.

A typical polymer system of our invention would contain a polymer containing approximately 40 to 80% poly(oxyethylene) and approximately 20 to 60% poly(oxypropylene). The total molecular weight of the polymer used in our invention is at a minimum about 7,000 and can go as high as 50,000 but preferably is in the range of 7,000 to 30,000; and x and y are any integers within the above constraints. Preferred polymers are those of the formula above where w = 2, namely the poloxamine polymer.

The aqueous drug delivery vehicle would contain from 10% to 50% by weight of the entire vehicle as polymer described above. The aqueous drug delivery vehicle would also contain the drug or therapeutic agent in addition to various additives such as acids or bases to adjust the pH of the composition, buffers to maintain the pH, preservatives to control bacterial contamination, other additives to provide for drug solubility and stability and formulation performance with purified water making up the remainder of the drug delivery vehicle.

## DETAILED DESCRIPTION OF THE INVENTION

The invention consists of a pharmaceutical composition or drug delivery system which is a clear physiological acceptable solution at room temperature or lower but which forms a semi-solid or gel when placed in the eye. The unique feature of this system is that both the gel transition temperature and/or the rigidity of the gel can be modified by adjustment of the pH and or ionic strength and polymer concentration.

The ability to change the sol-gel transition temperature by pH adjustment is a critical feature of the invention which overcomes many of the disadvantages of previous approaches. Also the sol-gel transition temperature can be modified somewhat by ionic strength adjustment.

An example of a drug delivery vehicle in accordance with this invention consists of an aqueous solution of, for example, a tetra substituted ethylene diamine block copolymer of poly(oxyethylene)-poly-

(oxypropylene) (where w = 2 in Formula I) in which the substitution at the nitrogen is to the poly-(oxypropylene) block and the polymer consists of about 40-80% as the poly(oxyethylene) unit and about 20-60% as the polypropylene unit and which has a total average molecular weight of 7,000 to 50,000 with a preferred range of 7,000-30,000. Such polymers are included in the polymers sold under the trademark "Tetronic®" polyols by BASF Wyandotte Corporation.

Other polymers where w = 3 to 6 (of Formula I) can be made according to methods known in the art (Block and Graft Copolymerization, Vol. 2 edited by R.J. Ceresa published by John Wiley and Sons, 1976) by using the appropriate initiators such as for example propylenediamine, butylenediamine, pentylenediamine and hexylenediamine.

The preferred polymers are those which form gels at a concentration range of 10 to 50% of the polymer to water.

A good example of a typical polymer used in the drug delivery system of our invention is Tetronic® 1307 which thermally gels over a concentration range of about 15% to 35% in water with gelling temperatures of about 30°C to 10°C at neutral pH. The gel strength at 35% concentration is much more rigid than at the 15% gel concentration. However, with a solution-gel transition temperature of about 10°C for the 35% solution any useful liquid product would have to be refrigerated below this temperature. A useful vehicle can be prepared however by modification of both concentration and pH. For example a 27% Tetronic® 1307 solution at neutral pH has a gel-sol transition temperature of about 16°C but at pH 4 (adjusted to such with HCl at 10°C) the transition temperature is about 25°C. The gel formed under these conditions meets the requirements of a fairly rigid gel which is a liquid at room temperature.

The effect of pH and polymer concentration on gelling temperature for Tetronic® 1307 is shown in Fig. 1. Thus, for example, at a concentration of polymer to water of 25% the gelling temperature is 19°C at pH 6 and increases to 26°C at pH 4.

For administration of the drug delivery system of our invention to various body cavities, that is to the rectum, urethra, nasal cavity, vagina, auditory meatus, oral cavity or buccal pouch; by injection either subcutaneously or intramuscularly; by application to the skin; and application to the eye as drops, the pH of the system can range from 2 to 9 with the preferred pH range being 4 to 8. The pH, concentration and gelling temperatures will vary for any individual polymer falling within the class covered in this invention and these factors can be determined by those skilled in the art in possession of this concept.

The pH of the drug delivery system is adjusted by adding the appropriate amount of a pharmaceutically acceptable acid or base to obtain the required pH. The acid or base can be any that are known to persons skilled in the art but are preferably hydrochloric acid or sodium hydroxide.

In general the drug delivery vehicle of the present invention will contain from about 0.01 to about 5% of the medicament or pharmaceutical, from about 10% to about 50% of the polymer and from 90% to about 45% water. In special situations, however, the amounts may be varied to increase or decrease the dosage schedule.

If desired, the drug delivery vehicle may also contain, in addition to the medicament, buffering agents and preservatives. Suitable water soluble preservatives which may be employed in the drug delivery vehicle are sodium bisulfite, sodium thiosulfate, ascorbate, benzalkonium chloride, chlorobutanol, thimerosal, phenylmercuric borate, parabens, benzyl alcohol and phenylethanol. These agents may be present in amounts of from 0.001 to 5% by weight and preferably 0.01 to 2%. Suitable water soluble buffering agents are alkali or alkali earth carbonates, phosphates, bicarbonates, citrates, borates, acetates, succinates and the like, such as sodium phosphate, citrate, borate, acetate, bicarbonate and carbonate. These agents may be present in amounts sufficient to maintain a pH of the system of between 2 to 9 and preferably 4 to 8. As such the buffering agent can be as much as 5% on a weight to weight basis of the total composition.

Another factor which can affect the gelling temperature of the drug delivery vehicle or pharmaceutical composition is its ionic strength and this can be varied by adding to the drug delivery vehicle a pharmaceutically acceptable salt, such as sodium chloride, potassium chloride or mixtures thereof or even suitable alkali metal salts such as sodium sulfate and the like. The effect of adding sodium chloride is to decrease the gelling temperature by about 3°C for a change of 0.2 molar in ionic strength.

Fortunately for a typical ophthalmic dosage the pH and ionic strength effects will help maintain the drug delivery system as a gel in the eye. For example, a 27% thermally gelling solution (thermogel) at pH 4 and low ionic strength (about 0) when in the eye will be bathed with pH 7.4 and isotonic lacrimal fluid which at the surface of the gel will act to lower the gel-sol transition temperature thus helping to maintain and insure a gelled formulation in the eye rather than having the drug delivery system liquefy and perhaps be eliminated rapidly from the eye through the lacrimal drainage system. Under most conditions of use when used subcutaneously or by intromuscular injection or when administered to a body cavity the body's pH and ionic strength will help maintain the drug delivery system as a gel.

A unique aspect of the gel which is formed in situ in the eye or preferably in the inferior cul-de-sac of the eye is its prolonged residence time compared to conventional ophthalmic solutions. The tear turnover usually dilutes and depletes the drug reservoir very rapidly in conventional solutions. The thermogel formulation dissolves more slowly and promotes an enhanced delivery of the dissolved or dispersed agent within it. This prolonged residence time leads to more effective levels of concentration of agent in the tear film. An example of this longer residence time of the drug in the tear film is shown in Figure 2. The two thermogel formulations show a higher concentration of timolol for an extended period of time than the conventional marketed product. A dose sparing effect on the total amount of drug or agent applied and greater therapeutic effectiveness can be achieved with the thermogel formulations due to higher concentration of agent in the tear film when the agent penetrates the eye if this is desired or within the tear film if penetration is not desired.

Any pharmaceutically active material or diagnostic agent may be delivered in the drug delivery system of this invention. Preferably the drug or pharmaceutical is water soluble although some drugs will show greater solubility in the polymer system than others. Also the drugs or diagnostic agents can be suspended in the polymer vehicle.

Suitable drugs or diagnostic agents which can be administered by the drug polymer delivery system of the present invention when applied ophthalmically are:

(1) antibacterial substances such as beta-lactam antibiotics, such as cefoxitin, n-formamidoyl thienamycin and other thienamycin derivatives, tetracyclines, chloramphenicol, neomycin, carbenicillin, colistin, penicillin G, polymyxin B, vancomycin, cefazolin, cephaloridine, chibrorifamycin, gramicidin, bacitracin, sulfonamides; aminoglycoside antibiotics such as gentamycin, kanamycin, amikacin, sisomicin and tobramycin; naladixic acid and analogs such as norfloxacin and the antimicrobial combination of fluealanine/ pentizidone; nitrofurazones, and the like;

(2) antihistaminics and decongestants such as pyrilamine, chlorpheniramine, tetrahydrazoline, antazoline, and the like;

(3) anti-inflammatories such as cortisone, hydrocortisone, hydrocortisone acetate, betamethasone, dexamethasone, dexamethasone sodium phosphate, prednisone, methylprednisolone, medrysone, fluorometholone, fluocortolone, prednisolone, prednisolone sodium phosphate, triamcinolone, indomethacin, sulindac, its salts and its corresponding sulfide, and the like;

(4) miotics and anticholinergics such as echothiophate, pilocarpine, physostigmine salicylate, diisopropylfluorophosphate, epinephrine, dipivolyl epinephrine, neostigmine, echothiopate iodide, demecarium bromide, carbachol, methacholine, bethanechol, and the like;

(5) mydriatics such as atropine, homatropine, scopolamine, hydroxyamphetamine, ephedrine, cocaine, tropicamide, phenylephrine, cyclopentolate, oxyphenonium, eucatropine, and the like; and other medicaments used in the treatment of eye conditions or diseases such as

(6) antiglaucoma drugs for example, timolol, especially as the maleate salt and R-timolol and a combination of timolol or R-timolol with pilocarpine. Also included are: epinephrine and epinephrine complex or prodrugs such as the bitartrate, borate, hydrochloride and dipivefrin derivatives and hyperosmotic agents such as glycerol, mannitol and urea;

(7) antiparasitic compounds and/or anti-protozoal compounds such as ivermectin; pyrimethamine, trisulfapyrimidine, clindamycin and corticosteroid preparations;

(8) antiviral effective compounds such as acyclovir, 5-iodo-2'-deoxyuridine (IDU), adenosine arabinoside (Ara-A), trifluorothymidine, and interferon and interferon inducing agents such as Poly I:C;

(9) carbonic anhydrase inhibitors such as acetazolamide, dichlorphenamide, 2-(p-hydroxyphenyl) thio-5-thiophenesulfonamide, 6-hydroxy-2-benzothiazolesulfonamide and 6-pivaloyloxy-2-benzothiazolesulfonamide;

(10) anti-fungal agents such as amphotericin B, nystatin, flucytosine, natamycin, and miconazole;

(11) anesthetic agents such as etidocaine cocaine, benoxinate, dibucaine hydrochloride, dyclonine hydrochloride, naepaine, phenacaine hydrochloride, piperocaine, proparacaine hydrochloride, tetracaine hydrochloride, hexylcaine, bupivacaine, lidocaine, mepivacaine and prilocaine;

(12) ophthalmic diagnostic agents such as

(a) those used to examine the retina and choride - sodium fluorescein;

(b) those used to examine the conjunctiva, cornea and lacrimal apparatus such as fluorescein and rose bengal; and

(c) those used to examine abnormal pupillary responses such as methacholine, cocaine, adrenaline, atropine, hydroxyamphetamine and pilocarpine;

(13) ophthalmic agents used as adjuncts in surgery such as alpha-chymotrypsin, and hyaluronidase;

(14) chelating agents such as ethylenediamine tetraacetate (EDTA) and deferoxamine;

(15) immunosuppressive agents and anti-metabolites such as methotrexate, cyclophosphamide, 6-mercaptopurine, and azathioprine; and

(16) combinations of the above such as antibiotic/ anti-inflammatory as in neomycin sulfate-dexamethasone sodium phosphate, concomitant anti-glaucoma therapy such as timolol maleate-aceclidine.

Typically as stated previously, the present liquid drug delivery device would contain from about 0.001 to about 5% of the medicament or pharmaceutical on a weight to weight basis. Thus, from one drop of the liquid composition which contains about 25 mg of solution, one would obtain about .0025 mg to about 1.25 mg of drug.

The particular drug or medicament used in the pharmaceutical composition of this invention is the type which a patent would require for pharmacological treatment of the condition from which said patient is suffering. For example, if the patient is suffering from glaucoma, the drug of choice would probably be timolol.

Also included in this invention when used ophthalmically is the use of the drug delivery device or pharmaceutical composition minus the active drug or medicament for the treatment of dry eye; restoration or maintenance of vaginal acidity; or a protective covering for the skin or for lubricating or emollient reasons so as to maintain proper mechanical function of the skin. All the ratios of components as described above would be satisfactory for the composition used for dry eye. For this use, one would administer the vehicle as needed at the desired pH.

Suitable drugs which can be administered in the drug delivery system of the present invention when administered to a body cavity are antibacterial substances such as β-lactam antibiotics, such as cefoxitin, n-formamidoyl thienamycin and other thienamycin derivatives, tetracyclines, chloramphenicol, neomycin, gramicidin, bacitracin, sulfonamides; aminoglycoside antibiotics such as gentamycin, kanamycin, amikacin, sisomicin and tobramycin; nalidixic acids and analogs such as norfloxacin and the antimicrobial combination of fludalanine/pentizidone; nitrofurazones, and the like; antihistaminics and decongestants such as pyrilamine, cholpheniramine, tetrahydrazoline, antazoline, and the like; anti-inflammatories such as cortisone, hydrocortisone, beta-methasone, dexamethasone, fluocortolone, prednisolone, triamcinolone, indomethacin, sulindac, its salts and its corresponding sulfide, and the like. Also included are antiparasitic compounds such as ivermectin; antiviral effective compounds such as acyclovir and interferon.

For otic use besides the antibacterials and anti-inflammatory drugs the use of local anesthetic is helpful in reducing the pain. Local anesthetics such as benzocaine, lidocaine, procaine and the like can be used.

For treatment of vaginal and urethral conditions requiring antifungal, amebecidal, trichomonacidal agent or antiprotozoals the following can be used such as polyoxyethylene nonylphenol, alkylaryl sulfonate, oxyquinoline sulfate, miconazole nitrate, sulfanilamide, candicidin, sulfisoxazole, nystatin, clotrimazole, metronidazole and the like and for antiprotozoals, chloramphenicol, chloroquine, trimethoprim, sulfamethoxazole and the like.

For use rectally the following suitable drugs can be administered by the drug polymer delivery system of the present invention:

(1) Analgesics such as aspirin, acetaminophen, diflunisal and the like;

(2) anesthetics such as lidocaine, procaine, benzocaine, xylocaine and the like;

(3) antiarthritics such as phenylbutazone, indomethacin, sulindac, dexamethasone, ibuprofen, allopurinol, oxyphenbutazone probenecid and the like;

(4) antiasthma drugs such as theophylline, ephedrine, beclomethasone dipropionate, epinephrine and the like;

(5) urinary tract disinfectives such as sulfamethoxazole, trimethoprim, nitrofurantoin, norfloxicin and the like;

(6) anticoagulants such as heparin, bishydroxy coumarin, warfarin and the like;

(7) anticonvulsants such as diphenylhydantoin, diazepam and the like;

(8) antidepressants such as amitriptyline, chlordiazepoxide, perphenazine, protriptyline, imipramine, doxepin and the like;

(9) antidiabetics such as insulin, tolbutamide, tolazamide, acetohexamide, chlorpropamide and the like

(10) antineoplastics such as adriamycin, flurouracil, methotrexate, asparaginase and the like;

(11) antipsychotics such as prochlorperazine, lithium carbonate, lithium citrate, thioridazine, molindone, fluphenazine, trifluoperazine, perphenazine, amitriptyline, triflupromazine and the like;

(12) antihypertensive such as spironolactone, methyldopa, hydralazine, clonidine, chlorothiazide, deserpidine, timolol, propranolol, metoprolol, prazosin hydrochloride, reserpine and the like; and

(13) muscle relaxants such as melphalan, danbrolene, cyclobenzaprine, methocarbamol, diazepam and the like.

(14) antiprotozoals such as chloramphenicol, chloroquine, trimethoprim and sulfamethoxazole.

Typically as stated previously, the present liquid drug delivery device would contain from about 0.001 to about 5% of the medicament or pharmaceutical on a weight to weight basis. Thus, from one gram of the liquid composition which is about 1 ml of solution, one would obtain about 0.1 mg to about 50 mg of drug.

Suitable drugs which can be administered in the drug delivery system of the present invention when administered by injection or subcutaneously to the body are antibacterial substances such as $\beta$-lactam antibiotics, such as cefoxitin, n-formamidoyl thienamycin and other thienamycin derivatives, tetracyclines, chloramphenicol, neomycin, gramicidin, bacitracin, sulfonamides; aminoglycoside antibiotics such as gentamycin, kanamycin, amikacin, sisomicin and tobramycin; nalidixic acids and analogs such as norfloxacin and the antimicrobial combination of fludalanine/pentizidone; nitrofurazones, and the like; antihistaminics and decongestants such as pyrilamine, cholpheniramine, tetrahydrazoline, antazoline, and the like; anti-inflammatories such as cortisone, hydrocortisone, beta-methasone, dexamethasone, fluocortolone, prednisolone, triamcinolone, indomethacin, sulindac, its salts and its corresponding sulfide, various peptide drugs such as insulin, somatostatin and analogs of those drugs,and the like. Also included are anti-parasitic compounds such as ivermectin; antiviral effective compounds such as acyclovir and interferon.

For use in subcutaneous or intramuscular injection the following suitable drugs can also be administered by the drug polymer delivery system of the present invention:

(1) Analgesics such as aspirin, acetaminophen, diflunisal and the like;

(2) anesthetics such as lidocaine, procaine, benzocaine, xylocaine and the like;

(3) antiarthritics such as phenylbutazone, indomethacin, sulindac, dexamethasone, ibuprofen, allopurinol, oxyphenbutazone probenecid and the like;

(4) antiasthma drugs such as theophylline, ephedrine, beclomethasone dipropionate, epinephrine and the like;

(5) urinary tract disinfectives such as sulfamethoxazole, trimethoprim, nitrofurantoin, norfloxacin and the like;

(6) anticoagulants such as heparin, bishydroxy coumarin, warfarin and the like;

(7) anticonvulsants such as diphenylhydantoin, diazepam and the like;

(8) antidepressants such as amitriptyline, chlordiazepoxide, perphenazine, protriptyline, imipramine, doxepin and the like;

(9) antidiabetics such as insulin, tolbutamide, somatostatin and its analogs, tolazanide, acetohexamide, chlorpropamide and the like

(10) antineoplastics such as adriamycin, flurouracil, methotrexate, asparaginase and the like;

(11) antipsychotics such as prochlorperazine, lithium carbonate, lithium citrate, thioridazine, molindone, fluphenazine, trifluoperazine, perphenazine, amitriptyline, triflupromazine and the like;

(12) antihypertensive such as spironolactone, methyldopa, hydralazine, clonidine, chlorothiazide, deserpidine, timolol, propranolol, metoprolol, prazosin hydrochloride, reserpine and the like; and

(13) muscle relaxants such as succinylcholine chloride, danbrolene, cyclobenzaprine, methocarbamol, diazepam and the like.

Typically as stated previously, the present liquid drug delivery device would contain from about 0.001 to about 5% of the medicament or pharmaceutical on a weight to weight basis. Thus, from one gram of the liquid composition which is about 1 ml of solution, one would obtain about 0.1 mg to about 50 mg of drug.

Suitable drugs which can be administered by the drug delivery system of the present invention when administered topically are antibacterial substances such as beta-lactam antibiotics, such as cefoxitin, n-formamidoyl thienamycin and other thienamycin derivatives, tetracyclines, chloramphenicol, neomycin, gramicidin, bacitracin, sulfonamides; aminoglycoside antibiotics such as gentamycin, kanamycin, amikacin, sisomicin and tobramycin; nalidixic acid and analogs such as norfloxacin and the antimicrobial combination of fludalanine/ pentizidone; nitrofurazones, anti-infectives such as iodine, chloramines, benzalkonium chloride, phenol and the like; anti-inflammatories such as cortisone, hydrocortisone, beta-methasone, dexamethasone, fluocortolone, prednisolone, triamcinolone, indomethacin, sulindac, its salts and its corresponding sulfide, and the like; anesthetics such as benzocaine, lidocaine, dibucaine and the like; analgesics such as methyl salicylate, menthol, camphor, methyl nicotinate, triethanolamine salicylate, glycol salicylate and salicylamide and the like; antifungal agents such as tolnaftate, undecylenic acid, salicylic acid, zinc undecylenate and thiabendazole and the like. Also included are antiparasitic compounds such as ivermectin, antiviral effective compounds such as idoxuridine, acyclovir and interferon.

Typically as stated previously, the present liquid drug delivery system would contain from about 0.001 to about 5% of the medicament or pharmaceutical on a weight to weight basis. Thus, from one gram of the liquid composition which is about 1 ml of solution, one would obtain about 0.1 mg to about 50 mg of medicament or pharmaceutical.

The preparation of the drug delivery systems are described below and the appropriate examples which

follow were all carried out according to this procedure. Since the polymer systems of this invention dissolve better at reduced temperatures, the preferred methods of solubilization are to add the required amount of polymer to the amount of water to be used. Generally, after wetting the polymer by shaking, the mixture is capped and placed in a cold chamber or in a thermostated container at about 0° C to 10° C to dissolve the polymer. The mixture can be stirred or shaken to bring about a more rapid solution of the polymer.

The drug substance or medicaments and various additives such as buffers, salts and preservatives are then added and dissolved. The final desired pH adjustment can be made by adding the appropriate acids or bases such as hydrochloric acid or sodium hydroxide to the drug delivery system.

When used in the eye the pharmaceutical composition will be administered as a fluid by any conventional means of delivering drop formulations to the eye such as by means of an eye-dropper or by using an Ocumeter®. Typically these formulations are intended to be administered into the inferior cul-de-sac of the eye. This can easily be accomplished by distending the lower lid from the eye and applying the drop within the sac and then releasing the lid.

When used to deliver drugs by injection, the pharmaceutical composition will be administered as a liquid by use of an appropriate syringe adapted with the appropriate delivery tube or needle.

When used to deliver drugs rectally, urethrally or vaginally, the pharmaceutical composition will be administered as a liquid by use of an appropriate syringe adapted with the appropriate delivery tube or needle. Also when used to treat conditions of the ear or nose the pharmaceutical composition will be administered as a liquid by any conventional means of delivering drop formulations such as by means of an eye-dropper or by using a squeeze bottle such as an Ocumeter®.

Any convenient method can be used to apply the pharmaceutical composition to the skin such as a bottle from which the solution is poured from. A ball roller can be used or a dropper-type application employing a rubber dropper.

## EXAMPLES

The following examples are illustrations and are not intended to be restrictive of the scope of the invention.

All percentages are given in (w/w) % and all pH measurements are for 10° C. In the animal experiments, 25 mg of each solution was administered to the inferior cul-de-sac of the eye.

### OPHTHALMIC

### EXAMPLE 1

The use of the polymer vehicle to deliver pilocarpine.

| | Solution 1 | Solution 2 | Solution 3 |
|---|---|---|---|
| Pilocarpine | 0.5% | 0.1% | 0.5% |
| Tetronic® 1307 | 27.0% | 27.0% | - |
| pH adjusted with HCl to | 4.0 | 4.0 | 4.0 |
| sufficient purified water to make | 100% | 100% | 100% |
| gel-sol transition temp. | 26°C | 26°C | - |

Pilocarpine is known to produce a miotic effect (constriction of the iris). In an experiment in rabbits the miotic effect of pilocarpine in the thermally gelling solutions 1 and 2 was compared with solution 3, a conventional liquid drop. The pupillary diameter change was measured over 3 hours. The results showed solution 1 had a larger area under the curve than 2 or 3 and that solutions 2 and 3 had about the same AUC.

The relative area under the curve measurements:

| Solution | Relative AUC |
|---|---|
| 1 | 1.3 |
| 2 | 0.9 |
| 3 | 1.0 |

These results indicate about a 5-fold reduction in pilocarpine concentration in the thermally gelling solution (solution 2) can produce a similar pharmacological response as a conventional drop (solution 3).

EXAMPLE 2

The use of the polymer vehicle to deliver timolol.

| | Solution 1 | Solution 2 |
|---|---|---|
| Timolol maleate | 0.68% | 0.68% |
| Tetronic® 1307 | 22.0% | 27.0% |
| pH adjusted with HCl to | 4 | 4 |
| sufficient water to make | 100% | 100% |
| gel-sol transition temperature | 30° | 26° |

The thermally gelling solutions 1 and 2 were compared to the commercially available Timoptic® solution (Timolol maleate 0.68%). The experiment involved measurement of the lacrimal fluid concentration of the drug with time. Even though a biological response is not measured, the effect of the thermally gelling solutions in maintaining higher drug concentrations for extended periods of time provides an indication of the response the drug should have. The solutions can be compared from the first-order decay rates and AUC.

| Solution | Half-life | Relative AUC |
|---|---|---|
| Solution 1 | 3.8 min. | 2.7 |
| Solution 2 | 13 min. | 3.3 |
| Timoptic® | 1.1 min. | 1 |

The thermally gelling solutions provide much slower elimination rates of the drug from eye.

EXAMPLE 3

The use of the polymer vehicle to deliver Norfloxacin.

| | Solution 1 | Solution 2 | Solution 3 | Solution 4 |
|---|---|---|---|---|
| Norfloxacin | 0.1% | 0.1% | 0.1% | 0.1% |
| Tetronic® 1307 | 22.0% | 27.0% | 32.0% | -- |
| pH adjusted with HCl to | 4 | 4 | 4 | 4 |
| sufficient purifind water to make | 100% | 100% | 100% | 100% |
| gel-sol transition temp. | 30°C | 26°C | 21°C | -- |

The concentration of norfloxacin was measured in the lacrimal fluid with time. The elimination rates of norfloxacin were slower for the thermally gelling solutions 1, 2 and 3 and produced larger AUC.

| Solution | Relative AUC |
|---|---|
| 1 | 2.2 |
| 2 | 1.7 |
| 3 | 1.9 |
| 4 | 1 |

EXAMPLE 4

| | Solution 1 | Solution 2 |
|---|---|---|
| Norfloxacin | 0.4% | 0.2% |
| Tetronic® 1307 | 27.0% | 0.0% |
| pH adjusted with HCl to | 4 | 4 |
| sufficient purified water to make | 100% | 100% |

The solutions in Example 4 can be used to demonstrate that a larger dose of drug can be administered in the thermally gelling solution without exceeding the saturation level of the drug. Solutions 1 and 2 in the rabbit eye both give initial concentrations of about 2 mg/ml even though solution 1 has twice the concentration. The slower release from such a thermally gelling solutions would be of value under such conditions.

EXAMPLE 5

| | |
|---|---|
| Dexamethasone | 0.05% |
| Tetronic® 1307 | 30.0% |
| Benzalkonium chloride | 0.02% |
| pH adjusted with HCl to 4 | |
| sufficient purified water to make | 100% |
| gel-sol transition temperature | 21° |

EXAMPLE 6

| | |
|---|---|
| Gentamycin Sulfate | 0.1% |
| Tetronic® 1307 | 25.0% |
| Benzalkonium chloride | 0.01% |
| Sodium chloride | 0.05% |
| pH adjusted with HCl to 4 | |
| sufficient purified water to make | 100% |
| gel-sol transition temperature | 26° |

EXAMPLE 7

| | |
|---|---|
| Chloramphenicol | 0.5% |
| Tetronic® 1508 | 20.0% |
| Sodium acetate | 0.3% |
| Benzalkonium chloride | 0.01% |
| pH adjusted with HCl to 5 | |
| sufficient purified water to make | 100% |
| gel-sol transition temperature | 27° |

If the pharmaceutical compositions of Examples 5-7 were compared with similar compositions but without the polymer, it would be expected that the compositions of Examples 5-7 would result in greater bioavailability and/or sustained concentrations of the drug in the eye.

Following the procedure of Examples 1-6 one can use an appropriate amount of the polymers listed below in place of the Tetronic® 1307 or Tetronic® 1508 polymer used in Examples 1-6 and 7.

Tetronic® 1107
Tetronic® 908
Tetronic® 707

Following the procedure of Examples 1-7 one can use an appropriate amount of the drugs or medicaments previously enumerated in this application in place of the drug or medicament used in Examples 1-7.

BODY CAVITY (ORAL)

EXAMPLE 1

The use of the polymer vehicle to deliver norfloxacin a broad spectrum antimicrobial compound.

| | Solution 1 | Solution 2 | Solution 3 |
|---|---|---|---|
| norfloxacin | 0.1% | 0.1% | 0.1% |
| Tetronic® 1307 | 22.0% | 27.0% | 32.0% |
| pH adjusted with HCl to | 4 | 4 | 4 |
| sufficient purified water to make | 100% | 100% | 100% |
| gel-sol transition temp. | 30°C | 26°C | 21°C |

All three solutions can be administered as described previously as liquids, however, solution 3 would require cooling to below 21 °C before use.

EXAMPLE 2

| | |
|---|---|
| Dexamethasone | 0.05% |
| Tetronic® 1307 | 30.0% |
| Benzalkonium chloride | 0.02% |
| pH adjusted with HCl to | 4 |
| sufficient purified water to make | 100% |
| gel-sol transition temperature | 21°C |

EXAMPLE 3

| | |
|---|---|
| Gentamycin sulfate | 0.1% |
| Tetronic® 1307 | 25.0% |
| Benzalkonium chloride | 0.01% |
| Sodium chloride | 0.05% |
| pH adjusted with HCl to | 4 |
| sufficient purified water to make | 100% |
| gel-sol transition temperature | 26°C |

EXAMPLE 4

| | |
|---|---|
| Chloramphenicol | 0.5% |
| Tetronic® 1508 | 20.0% |
| Sodium acetate | 0.3% |
| Benzalkonium chloride | 0.01% |
| pH adjusted with HCl to 5 | |
| sufficient purified water to | 100% |
| make gel-sol transition temperature | 27°C |

EXAMPLE 5

| | |
|---|---|
| Lidocaine | 5% |
| Tetronic® 1307 | 25% |
| Benzalkonium chloride | 0.01% |
| pH adjusted with HCl to 4 | |
| Sufficient purified water to | 100% |
| make gel-sol transition temperature | 32°C |

EXAMPLE 6

| | |
|---|---|
| Sodium acetate | 2% |
| Tetronic® 1307 | 21% |
| Benzalkonium chloride | 0.01% |
| pH adjusted with HCl to 5 | |
| sufficient purified water to | 100% |
| gel-sol transition temperature | 25°C |

EXAMPLE 7

| | Solution 1 | Solution 2 |
|---|---|---|
| Timolol maleate | 0.68% | 0.68% |
| Tetronic® 1307 | 22% | 27% |
| pH adjusted with HCl to | 4 | 4 |
| sufficient purified water | 100% | 100% |
| gel-sol transtion temperature | 30°C | 26°C |

If the pharmaceutioal compositions of Example 1-7 were compared with similar compositions but without the polymer, it would be expected that the compositions of Examples 1-7 would result in greater sustained concentrations of the drug at the site of administration.

Following the procedure of Examples 1-7 one can use an appropriate amount of the polymers listed below in place of the Tetronic® 1307 or Tetronic® 1508 polymer used in Examples 1-7.

Tetronic® 1107

Tetronic® 908

Tetronic® 707

Following the procedure of Examples 1-7 one can use an appropriate amount of the drugs previously enumerated in this application.

INJECTION

EXAMPLE 1

The use of the polymer vehicle to deliver norfloxacin a broad spectrum antimicrobial compound.

| | Solution 1 | Solution 2 | Solution 3 |
|---|---|---|---|
| Norfloxacin | 0.1% | 0.1% | 0.1% |
| Tetronic® 1307 | 22.0% | 27.0% | 32.0% |
| pH adjusted with HCl to | 4 | 4 | 4 |
| sufficient purified water to make | 100% | 100% | 100% |
| gel-sol transition temp. | 30°C | 26°C | 21°C |

All three solutions can be administered as described previously as liquids, however, solution 3 would require cooling to below 21 °C before use.

EXAMPLE 2

| | |
|---|---|
| Dexamethasone | 0.05% |
| Tetronic® 1307 | 30.0% |
| Benzalkonium chloride | 0.02% |
| pH adjusted with HCl to 4 | |
| sufficient purified water to make | 100% |
| gel-sol transition temperature | 21°C |

EXAMPLE 3

| | |
|---|---|
| Gentamycin sulfate | 0.1% |
| Tetronic® 1307 | 25.0% |
| Benzalkonium chloride | 0.01% |
| Sodium chloride | 0.05% |
| pH adjusted with HCl to 4 | |
| sufficient purified water to make | 100% |
| gel-sol transition temperature | 26°C |

EXAMPLE 4

| | |
|---|---|
| Chloramphenicol | 0.5% |
| Tetronic® 1508 | 20.0% |
| Sodium acetate | 0.3% |
| Benzalkonium chloride | 0.01% |
| pH adjusted with HCl to 5 | |
| sufficient purified water to | 100% |
| make gel-sol transition temperature | 27°C |

EXAMPLE 5

| | |
|---|---|
| Lidocaine | 5% |
| Tetronic® 1307 | 25% |
| Benzalkonium chloride | 0.01% |
| pH adjusted with HCl to 4 | |
| Sufficient purified water to | 100% |
| make gel-sol transition temperature | 32°C |

EXAMPLE 6

| | |
|---|---|
| Insulin | 0.2% |
| Tetronic® 1307 | 25% |
| Phenol | 0.2% |
| pH adjusted with HCl to 4.2 | |
| sufficient purified water to make | 100% |
| gel-sol transition temperature | 29°C |

EXAMPLE 7

| | Solution 1 | Solution 2 |
|---|---|---|
| Timolol maleate | 0.68% | 0.68% |
| Tetronic® 1307 | 22% | 27% |
| pH adjusted with HCl to 4 | | |
| sufficient purified water | 100% | 100% |
| to make gel-sol transtion temperature | 30°C | 26°C |

If the pharmaceutical compositions of Examples 1-7 were compared with similar compositions but without the polymer, it would be expected that the compositions of Examples 1-7 would result in greater

sustained concentrations of the drug at the site of administration.

Following the procedure of Examples 1-7 one can use an appropriate amount of the polymers listed below in place of the Tetronic® 1307 or Tetronic® 1508 polymer used in Examples 1-7.

Tetronic® 1107
Tetronic® 908
Tetronic® 707

Following the procedure of Examples 1-7 one can use an appropriate amount of the drugs previously enumerated in this application.

TOPICAL

EXAMPLE 1

The use of the polymer vehicle to deliver norfloxacin, a broad-spectrum antimicrobial compound.

| | Solution 1 | Solution 2 | Solution 3 |
|---|---|---|---|
| Norfloxacin | 0.1% | 0.1% | 0.1% |
| Tetronic® 1307 | 22.0% | 27.0% | 32.0% |
| pH adjusted with HCl to 4 | | | |
| sufficient purified water to make | 100% | 100% | 100% |
| gel-sol transition temp. | 30°C | 26°C | 21°C |

All three solutions can be administered as described previously as liquids, however, solution 3 would require cooling to below 21 °C before use.

EXAMPLE 2

| | |
|---|---|
| Dexamethasone | 0.05% |
| Tetronic® 1307 | 30.0% |
| Benzalkonium chloride | 0.02% |
| pH adjusted with HCl to 4 | |
| sufficient purified water to make | 100% |
| gel-sol transition temperature | 21°C |

EXAMPLE 3

| | |
|---|---|
| Gentamycin sulfate | 0.1% |
| Tetronic® 1307 | 25.0% |
| Benzalkonium chloride | 0.01% |
| Sodium chloride | 0.05% |
| pH adjusted with HCl to 4 | |
| sufficient purified water to make | 100% |
| gel-sol transition temperature | 26°C |

EXAMPLE 4

| | |
|---|---|
| Chloramphenicol | 0.5% |
| Tetronic® 1508 | 20.0% |
| Sodium acetate | 0.3% |
| Benzalkonium chloride | 0.01% |
| pH adjusted with HCl to 5 | |
| sufficient purified water to make | 100% |
| gel-sol transition temperature | 27°C |

EXAMPLE 5

| | |
|---|---|
| Lidocaine | 5.0% |
| Tetronic® 1307 | 25.0% |
| Benzalkonium chloride | 0.01% |
| pH adjusted with HCl to 4 | |
| sufficient purified water to make | 100% |
| gel-sol transition temperature | 32°C |

EXAMPLE 6

| | |
|---|---|
| Sodium acetate | 2.0% |
| Tetronic® 1307 | 21.0% |
| Benzalkonium chloride | 0.01% |
| pH adjusted with HCl to 5 | |
| sufficient purified water to make | 100% |
| gel-sol transition temperature | 25°C |

If the pharmaceutical compositions of Examples 1-6 were compared with similar compositions but without the polymer, it would be expected that the compositions of Examples 1-6 would result in greater sustained concentrations of the drug at the site of administration.

Following the procedure of Examples 1-6 one can use an appropriate amount of the polymers listed below in place of the Tetronic® 1307 or Tetronic® 1508 polymer used in Examples 1-6.
Tetronic® 1107
Tetronic® 908
Tetronic® 707

Following the procedure of Examples 1-6 one can use an appropriate amount of the drugs previously enumerated in this application.

## Claims

1. An aqueous pharmaceutical composition for administration in liquid form to a patient requiring pharmalogical treatment comprising on one hand : a mixture of :
   a. 10% to 50% by weight of a polymer of the formula

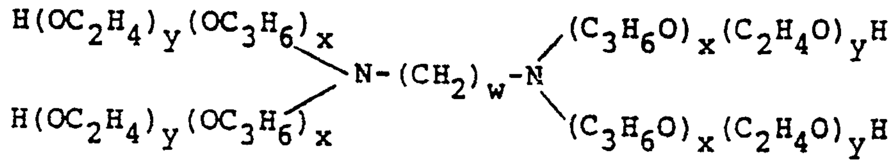

   wherein w is an integer from 2 to 6 containing approximately 40% to 80% poly(oxyethylene) and approximately 20 to 60% poly(oxypropylene) and having a molecular weight of 7,000 to 50,000; and x and y are any integers within the above constraints, and
   b. a pharmacologically effective amount of a pharmaceutical or diagnostic agent ; said mixture having a liquid gel transition temperature lower than the room temperature, and on the other hand :
   c. hydrochloric acid or sodium hydroxyde in sufficient quantity to adjust the liquid gel transition temperature to above room temperature, the pH of the composition ranging from 2 to 9.

2. An aqueous ophtalmic pharmaceutical composition according to claim 1 for treating an eye condition requiring pharmacological treatment wherein the pharmacologically effective drug is selected from the group consisting of antibacterial substances antihistamines and decongestants, anti-inflammatories, miotics and anticholinergics, mydriatics, antiglaucoma drugs, antiparasitics, antivirals, carbonic anhydrase inhibitors, antifungals, anesthetic agents, ophthalmic diagnostic agents, ophthalmic agents used as adjuvants in surgery, chelating agents, immuno-suppressive agents and anti-metabolites and combinations thereof.

3. A composition according to Claim 2 wherein the drug is selected from the group of compounds consisting of cefoxitin, n-formamidoyl thienamycin, tetracyclines, chloramphenicol, neomycin, carbenicillin, colistin, penicillin G, polymyxin B, vancomycin, cefazolin, cephaloridine, chibrorifamycin, gramicidin, bacitracin, sulfonamides, gentamycin, kanamycin, amikacin, sisomicin, tobramycin, naladixic acid, norfloxacin, combination of fluealanine/pentizidone, nitrofurazones; pyrilamine, chlorpheniramine, tetrahydrazoline, antazoline, cortisone, hydrocortisone, hydrocortisone acetate, betamethasone, dexamethasone, dexamethasone sodium phosphate, prednisone, methylprednisolone, medrysone, fluorometholone, fluocortolone, prednisolone, prednisolone sodium phosphate, triamcinolone, indomethacin, sulindac, echothiophate, pilocarpine, physostigmine salicylate, diisopropylfluorophosphate, epinephrine, dipivolyl epinephrine, neostigmine, echothiopate iodide, demecarium bromide, carbachol, methacholine, bethanechol, atropine, homatropine, scopolamine, hydroxyamphetamine, ephedrine, cocaine, tropicamide, phenylephrine, cyclopentolate, oxyphenonium, eucatropine, timolol, especially as the maleate salt and R-timolol and a combination of timolol or R-timolol with pilocarpine, epinephrine and epinephrine complex, bitartrate, borate, hydrochloride and dipivefrin derivatives, glycerol, mannitol, urea, ivermectin, pyrimethamine, trisulfapyrimidine, clindamycin, acyclovir, 5-iodo-2'-deoxyuridine (IDU), adenosine arabinoside (Ara-A), trifluorothymidine, interferon, Poly I:C, acetazolamide, dichlorphenamide, 2-(p-hydroxyphenyl)thio-5-thiophenesulfonamide, 6-hydroxy-2-benzothiazolesulfonamide, 6-pivaloyloxy-2-benzothiazole-sulfonamide, amphotericin B, nystatin, flucytosine, natamycin, miconazole, etidocaine cocaine, benoxinate, dibucaine hydrochloride, dyclonine hydrochloride, naepaine, phenacaine hydrochloride, piperocaine, proparacaine hydrochloride, tetracaine hydrochloride, hexylcaine, bupivacaine, lidocaine, mepivacaine, prilocaine, ophthalmic diagnostic agents, sodium

fluorescein, cornea, fluorescein, rose bengal, methacholine, cocaine, adrenaline, atropine, hydroxyamphetamine, pilocarpine, alpha-chymotrypsin, hyaluronidase, ethylenediamine tetraacetate (EDTA), deferoxamine, methotrexate, cyclophosphamide, 6-mercaptopurine, azathioprine, and combinations thereof.

**4.** A composition according to Claim 3 wherein the antiglaucoma is timolol maleate, R-timolol or a combination of timolol or R-timolol or pilocarpine; antibacterial is norfloxacin, chloramphenicol or cefoxitin; and the anti-inflammatory is dexamethasone, indomethacin or sulindac.

**5.** An injectable composition according to Claim 1 wherein the pharmacologically effective drug is selected from the group consisting of antibacterial substances, antihistamines and decongestants, anti-inflammatories, antiparasitics, antivirals, local anesthetics, antifungal, amebecidal, or trichomonocidal agents, analgesics, antiarthritics, antiasthmatics, anticoagulants, anticonvulsants. antidepressants, antidiabetics, antineoplastics, antipsychotics, antihypertensives and muscle relaxants.

**6.** A composition according to Claim 2 wherein the drug is selected from the group of compounds consisting of cefoxitin, n-formamidoyl thienamycin, tetracyclines, chloramphenicol, neomycin, gramicidin, bacitracin, sulfonamides, gentamycin, kanamycin, amikacin, sisomicin, tobramycin, nalidixic acids, norfloxacin, antimicrobial combination of fludalanine/pentizidone, nitrofurazones, pyrilamine, chlorpheniramine, tetrahydrazoline, antazoline, cortisone, hydrocortisone, beta-methasone, dexamethasone, fluocortolone, prednisolone, triamcinolone, indomethacin, sulindac, ivermectin, acyclovir, interferon, aspirin, acetaminophen, diflunisal, lidocaine, procaine, benzocaine, xylocaine, phenylbutazone, indomethacin, sulindac, dexamethasone, ibuprofen, allopurinol, oxyphenbutazone probenecid, theophylline, ephedrine, beclomethasone dipropionate, epinephrine, sulfamethoxazole, trimethoprim, nitrofurantoin, norfloxacin, heparin, bishydroxy coumarin, warfarin, diphenylhydantoin, diazepam, amitriptyline, chlordiazepoxide, perphenazine, protriptyline, imipramine, doxepin, insulin, tolbutamide, somatostatin and its analogs, tolazanide, acetohexamide, chlorpropamide, adriamycin, flurouracil, methotrexate, asparaginase, prochlorperazine, lithium carbonate, lithium citrate, thioridazine, molindone, fluphenazine, trifluoperazine, perphenazine, amitrlptyline, triflupromazine, spironolactone, methyldopa, hydralazine, clonidine, chlorothiazide, deserpidine, timolol, propranolol, metoprolol, prazosin hydrochloride, reserpine, succinylcholine chloride, danbrolene, cyclobenzaprine, methocarbamol and diazepam.

**7.** A composition according to Claim 5 wherein the antibacterial is gentamycin, norfloxacin or chloramphenicol; antidiabetic is insulin; anti-inflammatory is dexamethasone, indomethacin or sulindac; and anesthetic is lidocaine or benzocaine.

**8.** A topical or dermatological composition according to Claim 1 wherein the pharmacologically effective drug is selected from the group consisting of antibacterials, anti-infectives, anti-inflammatories, anesthetics, antifungals, antiparasitics and diagnostics.

**9.** A composition according to Claim 8 wherein the drug is selected from the group of compounds consisting of cefoxitin, n-formamidoyl thienamycin, tetracyclines, chloramphenicol, neomycin, gramicidin, bacitracin, sulfonamides, gentamycin, kanamycin, amikacin, sisomicin, tobramycin, nalidixic acid, norfloxacin, antimicrobial combination of fludalanine/pentizidone, nitrofurazones, iodine, chloramines, benzalkonium chloride, phenol, cortisone, hydrocortisone, beta-methasone, dexamethasone, fluocortolone, prednisolone, triamcinolone, indomethacin, sulindac, benzocaine, lidocaine, dibucaine, methyl salicylate, menthol, camphor, methyl nicotinate, triethanolamine salicylate, glycol salicylate, salicylamide, tolnaftate, undecylenic acid, salicylic acid, zinc undecylenate, thiabendazole, ivermectin, idoxuridine, acyclovir and interferon.

**10.** A composition according to Claim 8 wherein the antibacterial is norfloxacin, gentamycin or chloramphenicol; anesthetic is lidocaine or benzocaine; and the anti-inflammatory is dexamethasone, indomethacin or sulindac.

**11.** An aqueous pharmaceutical composition according to Claim 1 for administration to a body cavity to treat a condition requiring pharmacological treatment comprising

a. 10% to 50% by weight of a polymer of the formula:

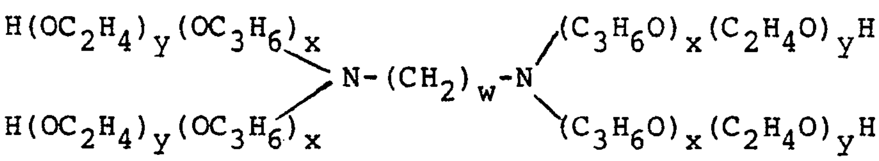

wherein w is an integer of from 2-6 containing approximately 40% to 80% poly(oxyethylene) and approximately 20 to 60% poly(oxypeopylene) and having a molecular weight of 7,000 to 50,000; and x and y are any integers within the above constraints,
b. a pharmacologically effective amount of drug selected from the group consisting of antibacterial substances, antihistamines and decongestants, anti-inflammatories, anti-parasitics, antiviral, local anesthetics, antifungal, amebecidal, or trichomonocidal agents, analgesics, antiarthritics, antiasthmatics, anticoagulants, anticonvulsants, antidepressants, antidiabetics, antineoplastics, antipsychotics, antihypertensives, and muscle relaxants and anti-protozoals; and
c. hydrochloric acid or sodium hydroxide in sufficient quantity to adjust the liquid gel transition temperature to above room temperature, the pH of the composition ranging from 2-9.

**12.** A composition according to Claim 11 wherein the drug is selected from the group of compounds consisting of cefoxitin, n-formamidoyl thienamycin, tetracyclines, chloramphenicol, neomycin, gramicidin, bacitracin, sulfonamides, gentamycin, kanamycin, amikacin, sisomicin, tobramycin, nalidixic acids, norfloxacin, antimicrobial combination of fludalanine/pentizidone, nitrofurazones, pyrilamine, cholpheniramine, tetrahydrazoline, antazoline, cortisone, hydrocortisone, beta-methasone, dexamethasone, fluocortolone, prednisolone, triamcinolone, indomethacin, sulindac, ivermectin, acyclovir, interferon, benzocaine, lidocaine, procaine, polyoxyethylene nonylphenol, alkylaryl sulfonate, oxyquinoline sulfate, miconazole nitrate, sulfanilamide, candicidin, sulfisoxazole, nystatin, clotrimazole, metronidazole, chloramphenicol, chloroquine, trimethoprim, sulfamethoxazole, aspirin, acetaminophen, diflunisal, lidocaine, procaine, benzocaine, xylocaine, phenylbutazone, indomethacin, sulindac, dexamethasone, ibuprofen, allopurinol, oxyphenbutazone probenecid, theophylline, ephedrine, beclomethasone dipropionate, epinephrine, sulfamethoxazole, trimethoprim, nitrofurantoin, norfloxicin, heparin, bishydroxy coumarin, warfarin, diphenylhydantoin, diazepam, amitriptyline, chlordiazepoxide, perphenazine, protriptyline, imipramine, doxepin, insulin, tolbutamide, tolazamide, acetohexamide, chlorpropamide, adriamycin, flurouracil, methotrexate, asparaginase, prochlorperazine, lithium carbonate, lithium citrate, thioridazine, molindone, fluphenazine, trifluoperazine, perphenazine, amitriptyline, triflupromazine, spironolactone, methyldopa, hydralazine, clonidine, chlorothiazide, deserpidine, timolol, propranolol, metoprolol, prazosin hydrochloride, reserpine, melphalan, danbrolene, cyclobenzaprine, methocarbamol, diazepam, chloramphenicol, chloroquine, trimethoprim and sulfamethoxazole.

**13.** A composition according to Claim 11 wherein the antihypertensive is timolol, methyldopa or hydrochlorothiazide; antibacterial is norfloxacin, chloramphenicol or gentamycin; anti-inflammatory is dexamethasone, indomethacin or sulindac; and anesthetic is lidocaine or benzocaine.

**14.** A composition according to Claim 1 wherein the polymer is one wherein W is 2.

**15.** A composition according to Claim 1 wherein the polymer is Tetronic® 1307.

**16.** The composition of Claim 1 wherein the gel-sol transition temperature of the composition is room temperature or below and said composition is liquid at this temperature.

**17.** A composition of Claim 1 further comprising at least one pharmaceutically acceptable buffering agent, salt and preservative.

## Revendications

**1.** Composition pharmaceutique agueuse pour l'administration sous une forme liquide à un patient nécessitant un traitement pharmacologique, comprenant d'une part : un mélange de :
a. 10 à 50 % en poids d'un polymère de formule

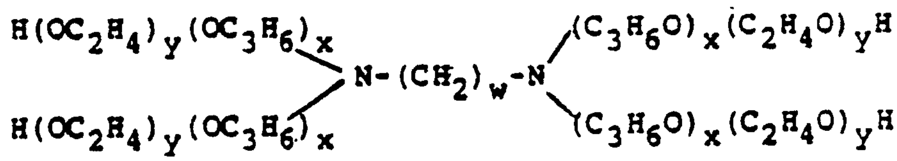

dans laquelle w est un entier de 2 à 6, contenant environ 40 % à 80 % de poly(oxyéthylène) et environ 20 à 60 % de poly(oxypropylène) et ayant un poids moléculaire de 7 000 à 50 000 ; et x et y sont des entiers quelconques dans les limites ci-dessus, et

b. une quantité pharmacologiquement efficace d'un agent pharmaceutique ou de diagnostic ;

ce mélange ayant une température de transition liquide-gel inférieure à la température ordinaire, et d'autre part :

c. de l'acide chlorhydrique ou de l'hydroxyde de sodium en une quantité suffisante pour ajuster la température de transition liquide-gel au-dessus de la température ordinaire,

le pH de la composition se situant entre 2 et 9.

2. Composition pharmaceutique ophtalmique aqueuse selon la revendication 1 pour le traitement d'une affection oculaire nécessitant un traitement pharmacologique, dans laquelle le médicament pharmacologiquement actif est choisi dans le groupe constitué par les substances antibactériennes, les antihistaminiques et les décongestionnants, les anti-inflammatoires, les myotiques et les anticholinergiques, les mydriatiques, les médicaments antiglaucomateux, les antiparasitaires, les antiviraux, les inhibiteurs de l'anhydrase carbonique, les antifongiques, les agents anesthésiques, les agents de diagnostic ophtalmique, les agents ophtalmiques utilisés comme appoints en chirurgie, les agents chélatants, les agents immunosuppresseurs et les antimétabolites, ainsi que leurs combinaisons.

3. Composition selon la revendication 2, dans laquelle le médicament est choisi dans le groupe des composés constitués par la céfoxitine, la N-formamidoylthiénamycine, les tétracyclines, le chloramphénicol, la néomycine, la carbénicilline, la colistine, la pénicilline G, la polymyxine B, la vancomycine, la céfazoline, la céfaloridine, la chibrorifamycine, la gramicidine, la bacitracine, les sulfamides, la gentamycine, la kanamycine, l'amikacine, la sisomicine, la tobramycine, l'acide nalidixique, la norfloxacine, l'association fludalanine/pentizidone, les nitrofurazones, la pyrilamine, la chlorphéniramine, la tétrahydrazoline, l'antazoline, la cortisone, l'hydrocortisone, l'acétate d'hydrocortisone, la bétaméthasone, la dexaméthasone, le dexaméthasone-phosphate sodique, la prednisolone, la méthylprednisolone, la médrysone, la fluorométholone, la fluocortolone, la prednisolone, le prednisolone-phosphate sodique, la triamcinolone, l'indométhacine, le sulindac, l'échothiopate, la pilocarpine, le salicylate de physostigmine, le fluorophosphate de diisopropyle, l'épinéphrine, la dipivalylépinéphrine, la néostigmine, l'iodure d'échothiopate, le bromure de démécarium, le carbachol, la méthacholine, le béthanechol, l'atropine, l'homatropine, la scopolamine, l'hydroxyamphétamine, l'éphédrine, la cocaïne, le tropicamide, la phényléphrine, le cyclopentolate, l'oxyphénonium, l'eucatropine, le timolol, en particulier le maléate et le R-timolol et une association de timolol ou de R-timolol et de pilocarpine, l'épinéphrine et un complexe, bitartrate, borate ou chlorhydrate d'épinéphrine et les dérivés de dipivéfrine, le glycérol, le mannitol, l'urée, l'ivermectine, la pyriméthamine, la trisulfapyrimidine, la clindamycine, l'acyclovir, la 5-iodo-2'-désoxyuridine (IDU), l'adénosine arabinoside (Ara-A), la trifluorothymidine, l'interféron, le Poly I:C, l'acétazolamide, le dichlorphénamide, le 2-(p-hydroxyphényl)thio-5-thiophènesulfonamide, le 6-hydroxy-2-benzothiazolesulfonamide, le 6-pivaloyloxy-2-benzothiazolesulfonamide, l'amphotéricine B, la nystatine, la flucytosine, la natamycine, le miconazole, l'étidocaïne, la cocaïne, le bénoxinate, le chlorhydrate de dibucaïne, le chlorhydrate de dyclonine, la naépaïne, le chlorhydrate de phénacaïne, la pipérocaïne, le chlorhydrate de proparacaïne, le chlorhydrate de tétracaïne, l'hexylcaïne, la bupivacaïne, la lidocaïne, la mépivacaïne, la prilocaïne, des agents de diagnostic ophtalmique, la fluorescéine sodique, la fluorescéine, le rose bengale, la méthacholine, la cocaïne, l'adrénaline, l'atropine, l'hydroxyamphétamine, la pilocarpine, l'$\alpha$-chymotrypsine, l'hyaluronidase, l'éthylènediaminetétraacétate (EDTA), la déféroxamine, le méthotrexate, le cyclophosphamide, la 6-mercaptopurine, l'azathioprine et leurs combinaisons.

4. Composition selon la revendication 3, dans laquelle l'agent antiglaucomateux est le maléate de timolol, le R-timolol ou une association de timolol ou de R-timolol et de pilocarpine ; l'agent antibactérien est la norfloxacine, le chloramphénicol ou la céfoxitine ; et l'agent anti-inflammatoire est la dexaméthasone, l'indométhacine ou le sulindac.

5. Composition injectable selon la revendication 1, dans laquelle le médicament pharmacologiquement efficace est choisi dans le groupe constitué par les substances antibactériennes, les antihistaminiques et les décongestionnants, les anti-inflammatoires, les antiparasitaires, les antiviraux, les anesthésiques locaux, les antifongiques, les agents amoebicides ou les agents trichomonacides, les analgésiques, les antiarthritiques, les antiasthmatiques, les anticoagulants, les anticonvulsivants, les antidépresseurs, les antidiabétiques, les antinéoplasiques, les antipsychotiques, les antihypertenseurs et les myorelaxants.

6. Composition selon la revendication 2, dans laquelle le médicament est choisi dans le groupe de composés constitué par la céfoxitine, la N-formamidoylthiénamycine, les tétracyclines, le chloramphénicol, la néomycine, la gramicidine, la bacitracine, les sulfamides, la gentamycine, le kanamycine, l'amikacine, la sisomicine, la tobramycine, l'acide nalidixique, la norfloxacine, l'association antimicrobienne fludalanine/pentizidone, les nitrofurazones, la pyrilamine, la chlorphéniramine, la tétrahydrazoline, l'antazoline, la cortisone, l'hydrocortisone, la bétaméthasone, la dexaméthasone, la fluocortolone, le prednisolone, la triamcinolone, l'indométhacine, le sulindac, l'ivermectine, l'acyclovir, l'interféron, l'aspirine, l'acétaminophène, le diflunisal, la lidocaïne, la procaïne, la benzocaïne, la xylocaïne, la phénylbutazone, l'indométhacine, le sulindac, la dexaméthasone, l'ibuprofène, l'allopurinol, l'oxyphenbutazone, le probénécide, la théophylline, l'éphédrine, le dipropionate de béclométhasone, l'épinéphrine, le sulfaméthoxazole, le triméthoprime, la nitrofurantoïne, la norfloxacine, l'héparine, la bishydroxycoumarine, la warfarine, la diphénylhydantoïne, le diazépam, l'amitriptyline, le chlordiazépoxide, la perphénazine, la protriptyline, l'imipramine, la doxépine, l'insuline, le tolbutamide, la somatostatine et ses analogues, le tolazanide, l'acétohexamide, le chlorpropamide, l'adriamycine, le fluoro-uracile, le méthotrexate, l'asparaginase, la prochlorpérazine, le carbonate de lithium, le citrate de lithium, la thioridazine, la molindone, la fluphénazine, la trifluopérazine, la perphénazine, l'amitriptyline, la triflupromazine, la spironolactone, la méthyldopa, l'hydralazine, la clonidine, le chlorothiazide, la déserpidine, le timolol, le propranolol, le métoprolol, le chlorhydrate de prazosine, la réserpine, le chlorure de succinylcholine, le danbrolène, la cyclobenzaprine, le méthocarbamol et le diazépam.

7. Composition selon la revendication 5, dans laquelle l'agent antibactérien est la gentamycine, la norfloxacine ou le chloramphénicol ; l'agent antidiabétique est l'insuline ; l'agent anti-inflammatoire est la dexaméthasone, l'indométhacine ou le sulindac ; et l'anesthésique est la lidocaïne ou la benzocaïne.

8. Composition locale ou dermatologique selon la revendication 1, dans laquelle le médicament pharmacologiquement efficace est choisi dans le groupe constitué par les antibactériens, les anti-infectieux, les anti-inflammatoires, les anesthésiques, les antifongiques, les antiparasitaires et les agents de diagnostic.

9. Composition selon la revendication 8, dans laquelle le médicament est choisi dans le groupe des composés constitués par la céfoxitine, la N-formamidoylthiénamycine, les tétracyclines, le chloramphénicol, la néomycine, la gramicidine, la bacitracine, les sulfamides, la gentamycine, la kanamycine, l'amikacine, la sisomicine, la tobramycine, l'acide nalidixique, la norfloxacine, une association antimicrobienne de fludalanine/pentizidone, les nitrofurazones, l'iode, les chloramines, le chlorure de benzalkonium, le phénol, la cortisone, l'hydrocortisone, la bétaméthasone, la dexaméthasone, la fluocortolone, la prednisolone, la triamcinolone, l'indométhacine, le sulindac, la benzocaïne, la lidocaïne, la dibucaïne, le salicylate de méthyle, le menthol, le camphre, le nicotinate de méthyle, le salicylate de triéthanolamine, le salicylate de glycol, le salicylamide, le tolnaftate, l'acide undécylénique, l'acide salicylique, l'undécylénate de zinc, le thiabendazole, l'ivermectine, l'idoxuridine, l'acyclovir et l'interféron.

10. Composition selon la revendication 8, dans laquelle l'agent antibactérien est la norfloxacine, la gentamycine ou le chloramphénicol ; l'agent anesthésique est la lidocaïne ou la benzocaïne ; et l'agent anti-inflammatoire est la dexaméthasone, l'indométhacine ou le sulindac.

11. Composition pharmaceutique agueuse selon la revendication 1 pour l'administration à une cavité de l'organisme pour traiter une affection nécessitant un traitement pharmacologique comprenant
   a. 10 à 50 % en poids d'un polymère de formule

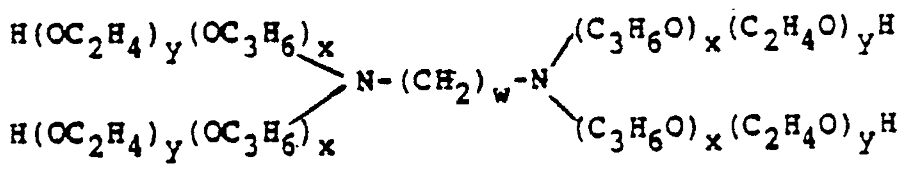

dans laquelle w est un entier de 2 à 6, contenant environ 40 % à 80 % de poly(oxyéthylène) et environ 20 à 60 % de poly(oxypropylène) et ayant un poids moléculaire de 7 000 à 50 000 ; et x et y sont des entiers quelconques dans les limites ci-dessus,

b. une quantité pharmacologiquement efficace d'un médicament choisi dans le groupe constitué par les substances antibactériennes, les antihistaminiques et les décongestionnants, les anti-inflammatoires, les antiparasitaires, les antiviraux, les anesthésiques locaux, les antifongiques, les amoebicides ou les agents trichomonacides, les analgésiques, les antiarthritiques, les antiasthmatiques, les anticoagulants, les anticonvulsivants, les antidépresseurs, les antidiabétiques, les antinéoplasiques, les antipsychotiques, les antihypertenseurs et les myorelaxants et les antiprotozoaires ; et

c. de l'acide chlorhydrique ou de l'hydroxyde de sodium en une quantité suffisante pour ajuster la température de transition liquide-gel au-dessus de la température ordinaire,

le pH de la composition se situant entre 2 et 9.

**12.** Composition selon la revendication 11, dans laquelle le médicament est choisi dans le groupe des composés constitués par la céfoxitine, la N-formamidoylthiénamycine, les tétracyclines, le chloramphénicol, la néomycine, la gramicidine, la bacitracine, les sulfamides, la gentamycine, la kanamycine, l'amikacine, la sisomicine, la tobramycine, l'acide nalidixique, la norfloxacine, une association antimicrobienne de fludalanine/pentizidone, les nitrofurazones, la pyrilamine, la chlorphéniramine, la tétrahydrazoline, l'antazoline, la cortisone, l'hydrocortisone, la bétaméthasone, la dexaméthasone, la fluocortolone, le prednisolone, la triamcinolone, l'indométhacine, le sulindac, l'ivermectine, l'acyclovir, l'interféron, la benzocaïne, la lidocaïne, la procaïne, le polyoxyéthylènenonylphénol, un alkylaryl sulfonate, le sulfate d'oxyquinoléine, le nitrate de miconazole, le sulfanilamide, la candicidine, le sulfisoxazole, la nystatine, le clotrimazole, le métronidazole, le chloramphénicol, la chloroquine, le triméthoprime, le sulfaméthoxazole, l'aspirine, l'acétaminophène, le diflunisal, la lidocaïne, la procaïne, la benzocaïne, la xylocaïne, la phénylbutazone, l'indométhacine, le sulindac, la dexaméthasone, l'ibuprofène, l'allopurinol, l'oxyphenbutazone, le probénécide, la théophylline, l'éphédrine, le dipropionate de béclométhasone, l'épinéphrine, le sulfaméthoxazole, le triméthoprime, la nitrofurantoïne, la norfloxicine, l'héparine, la bishydroxycoumarine, la warfarine, la diphénylhydantoïne, le diazépam, l'amitriptyline, le chlordiazépoxide, la perphénazine, la protriptyline, l'imipramine, la doxépine, l'insuline, le tolbutamide, le tolazamide, l'acétohexamide, le chlorpropramide, l'adriamycine, le fluoro-uracile, le méthotrexate, l'asparaginase, la prochlorperazine, le carbonate de lithium, le citrate de lithium, la thioridazine, la molindone, la fluphénazine, la trifluopérazine, la perphénazine, l'amitriptyline, la triflupromazine, la spironolactone, la méthyldopa, l'hydralazine, la clonidine, le chlorothiazide, la déserpidine, le timolol, le propranolol, le métoprolol, le chlorhydrate de prazosine, la réserpine, le melphalan, le danbrolène, la cyclobenzaprine, le méthocarbamol, le diazépam, le chloramphénicol, la chloroquine, le triméthoprime et le sulfaméthoxazole.

**13.** Composition selon la revendication 11, dans laquelle l'agent antihypertenseur est le timolol, la méthyldopa ou l'hydrochlorothiazide ; l'agent antibactérien est la norfloxacine, le chloramphénicol ou la gentamycine ; l'agent anti-inflammatoire est la dexaméthasone, l'indométhacine ou le sulindac ; et l'agent anesthésique est la lidocaïne ou la benzocaïne.

**14.** Composition selon la revendication 1 dans le polymère de laquelle w est 2.

**15.** Composition selon la revendication 1, dans laquelle le polymère est le Tetronic® 1307.

**16.** Composition selon la revendication 1, dans laquelle la température de transition gel-sol de la composition est égale ou inférieure à la température ordinaire et ladite composition est liquide à cette température.

**17.** Composition selon la revendication 1 comprenant de plus au moins un agent tampon, sel et conservateur pharmaceutiquement acceptables.

**Patentansprüche**

1. Wässrige pharmazeutische Zusammensetzung zur Verabreichung in flüssiger Form an einen Patienten, der der pharmakologschen Behandlung bedarf, welche einerseits eine Mischung aus
   a. 10 bis 50 Gew.-% eines Polymers der Formel

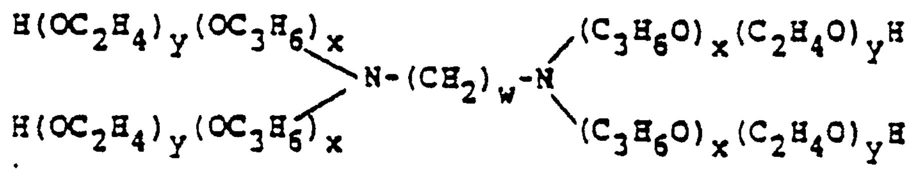

   worin w eine ganze Zahl von 2 bis 6 ist, das ungefähr 40 bis 80 % Poly(oxyethylen) und ungefähr 20 bis 60 % Poly(oxypropylen) enthält und ein Molekulargewicht von 7 000 bis 50 000 hat und worin x und y beliebige ganze Zahlen innerhalb der vorstehenden Beschränkungen sind, sowie
   b. eine pharmakologisch wirksame Menge eines pharmazeutischen oder diagnostischen Mittels, wobei die Mischung eine Flüssig-Gel-Übergangstemperatur von weniger als Raumtemperatur hat, und andererseits
   c. Salzsäure oder Natriumhydroxid in einer ausreichenden Menge, um die Flüssig-Gel-Übergangstemperatur auf über Raumtemperatur einzustellen, wobei der pH-Wert der Zusammensetzung im Bereich von 2 bis 9 liegt, umfaßt.

2. Wässrige ophthalmische pharmazeutische Zusammensetzung nach Anspruch 1 zur Behandlung eines die pharmakologische Behandlung erfordernden Augenzustands, worin das pharmakologisch wirksame Medikament aus der aus antibakteriellen Substanzen, Antihistaminen und Dekongestantien, entzündungshemmenden Mitteln, Miotica und Anticholinergica, Mydriatica, Antiglaukommedikamenten, Antiparasitenmitteln, Antivirusmitteln, Carbonsäureanhydraseinhibitoren, Antipilzmitteln, anästhetischen Mitteln, ophthalmischen diagnostischen Mitteln, als Adjuvantien in der Chirurgie verwandten ophthalmischen Mitteln, chelierenden Mitteln, immunsuppressiven Mitteln und Antimetaboliten sowie Kombinationen davon bestehenden Gruppe ausgewählt ist.

3. Zusammensetzung nach Anspruch 2, worin das Medikament aus der aus Cefoxitin, n-Formamidoylthienamycin, Tetracyclinen, Chloramphenicol, Neomycin, Carbenicillin, Colistin, Penicillin G, Polymyxin B, Vancomycin, Cefazolin, Cephaloridin, Chibrorifamycin, Gramicidin, Bacitracin, Sulfonamiden, Gentamycin, Kanamycin, Amikacin, Sisomicin, Tobramycin, Naladixinsäure, Norfloxacin, Kombinationen von Fludalanin/Pentizidon, Nitrofurazonen, Pyrilamin, Chlorpheniramin, Tetrahydrazolin, Antazolin, Cortison, Hydrocortison, Hydrocortisonacetat, Betamethason, Dexamethason, Dexamethason-Natriumphosphat, Prednison, Methylprednisolon, Medryson, Fluormetholon, Fluorcortolon, Prednisolon, Prednisolon-Natriumphosphat, Triamcinolon, Indomethacin, Sulindac, Echothiophat, Pilocarpin, Physostigminsalicylat, Diisopropylfluorphosphat, Epinephrin, Dipivolylepinephrin, Neostigmin, Echothiophatjodid, Demecariumbromid, Carbachol, Methacholin, Bethanechol, Atropin, Homatropin, Scopolamin, Hydroxyamphetamin, Ephedrin, Kokain, Tropicamid, Phenylephrin, Cyclopentolat, Oxyphenonium, Eucatropin, Timolol, insbesondere als Maleatsalz und R-Timolol und als Kombination von Timolol oder R-Timolol mit Pilocarpin, Epinephrin und Epinephrinkomplex, -bitartrat, -borat, -hydrochlorid und -dipivefrinderivate, Glycerol, Mannitol, Harnstoff, Ivermectin, Pyrimethamin, Trisulfapyrimidin, Clindamycin, Acyclovir, 5-Jod-2'-deoxyuridin (IDU), Adenosinarabinosid (Ara-A), Trifluorthymidin, Interferon, Poly-I:C, Acetazolamid, Dichlorphenamid, 2-(p-Hydroxyphenyl)thio-5-thiophensulfonamid, 6-Hydroxy-2-benzothiazolsulfonamid, 6-Pivaloyloxy-2-benzothiazolsulfonamid, Amphotericin B, Nystatin, Flucytosin, Natamycin, Miconazol, Etidocainkokain, Benoxinat, Dibucainhydrochlorid, Dycloninhydrochlorid, Naepain, Phenacainhydrochlorid, Piperocain, Proparacainhydrochlorid, Tetracainhydrochlorid, Hexylcain, Bupivacain, Lidocain, Mepivacain, Prilocain, ophthalmischen diagnostischen Mitteln, Natriumfluorescein, Hornhaut, Fluorescein, Bengalrosa, Methacholin, Kokain, Adrenalin, Atropin, Hydroxyamphetamin, Pilocarpin, Alpha-Chymotrypsin, Hyaluronidase, Ethylendiamintetraacetat (EDTA), Deferoxamin, Methotrexat, Cyclophosphamid, 6-Mercaptopurin, Azathioprin und Kombinationen davon bestehenden Verbindungsgruppe ausgewählt ist.

4. Zusammensetzung nach Anspruch 3, worin das Antiglaukommittel Timololmaleat, R-Timolol oder eine Kombination von Timolol oder R-Timolol oder Pilocarpin ist, das antibakterielle Mittel Norfloxacin,

Chloramphenicol oder Cefoxitin ist und das entzündungshemmende Mittel Dexamethason, Indomethacin oder Sulindac ist.

5. Injizierbare Zusammensetzung nach Anspruch 1, worin das pharmakologisch wirksame Medikament aus der aus antibakteriellen Substanzen, Antihistaminica und Dekongestantien, entzündungshemmenden Mitteln, Antiparasitenmitteln, Antivirusmitteln, lokalen Anästhetica, Antipilz-, Amebecidal- oder Trichomonocidalmitteln, Analgetica, Antiarthritica, Antiasthmatica, Antikoagulantien, Antikonvulsantien, Antidepressiva, Antidiabetica, Antineoplastica, Antipsychotica, Antihypertensica und Muskelrelaxantien bestehenden Gruppe ausgewählt ist.

6. Zusammensetzung nach Anspruch 2, worin das Medikament aus der aus Cefoxitin, n-Formamidoylthienamycin, Tetracyclinen, Chloramphenicol, Neomycin, Gramicidin, Bacitracin, Sulfonamiden, Gentamycin, Kanamycin, Amikacin, Sisomicin, Tobramycin, Nalidixinsäuren, Norfloxacin, einer antimikrobiellen Kombination von Fludalanin/Pentizidon, Nitrofurazonen, Pyrilamin, Cholpheniramin, Tetrahydrazolin, Antazolin, Cortison, Hydrocortison, Beta-Methason, Dexamethason, Fluocortolon, Prednisolon, Triamcinolon, Indomethacin, Sulindac, Ivermectin, Acyclovir, Interferon, Aspirin, Acetaminophen, Diflunisal, Lidocain, Procain, Benzocain, Xylocain, Phenylbutazon, Indomethacin, Sulindac, Dexamethason, Ibuprofen, Allopurinol, Oxyphenbutazonprobenecid, Theophyllin, Ephedrin, Beclomethasondipropionat, Epinephrin, Sulfamethoxazol, Trimethoprim, Nitrofurantoin, Norfloxacin, Heparin, Bishydroxycoumarin, Warfarin, Diphenylhydantoin, Diazepam, Amitriptylin, Chlordiazepoxid, Perphenazin, Protriptylin, Imipramin, Doxepin, Insulin, Tolbutamid, Somatostatin und seinen Analogen, Tolazanid, Acetohexamid, Chlorpropamid, Adriamycin, Fluoruracil, Methotrexat, Asparaginase, Prochlorperazin, Lithiumcarbonat, Lithiumcitrat, Thioridazin, Molindon, Fluphenazin, Trifluoperazin, Perphenazin, Amitriptylin, Triflupromazin, Spironolacton, Methyldopa, Hydralazin, Clonidin, Chlorothiazid, Deserpidin, Timolol, Propranolol, Metoprolol, Prazosinhydrochlorid, Reserpin, Succinylcholinchlorid, Danbrolen, Cyclobenzaprin, Methocarbamol und Diazepam bestehenden Verbindungsgruppe ausgewählt ist.

7. Zusammensetzung nach Anspruch 5, worin das antibakterielle Mittel Gentamycin, Norfloxacin oder Chloramphenicol ist, das Antidiabeticum Insulin ist, das entzündungshemmende Mittel Dexamethason, Indomethacin oder Sulindac ist und das Anästheticum Lidocain oder Benzocain ist.

8. Topische oder dermatologische Zusammensetzung nach Anspruch 1, worin das pharmakologisch wirksame Medikament aus der aus antibakteriellen Mitteln, infektionshemmenden Mitteln, entzündungshemmenden Mitteln, Anästhetica, Antipilzmitteln, Antiparasitenmitteln und Diagnostica bestehenden Gruppe ausgewählt ist.

9. Zusammensetzung nach Anspruch 8, worin das Mittel aus der aus Cefoxitin, n-Formamidoylthienamycin, Tetracyclinen, Chloramphenicol, Neomycin, Gramicidin, Bacitracin, Sulfonamiden, Gentamycin, Kanamycin, Amikacin, Sisomicin, Tobramycin, Nalidixinsäure, Norfloxacin, einer antimikrobiellen Kombination von Fludalanin/Pentizidon, Nitrofurazonen, Jod, Chloraminen, Benzalkoniumchlorid, Phenol, Cortison, Hydrocortison, Beta-Methason, Dexamethason, Fluorcortolon, Prednisolon, Triamcinolon, Indomethacin, Sulindac, Benzocain, Lidocain, Dibucain, Methylsalicylat, Menthol, Campfer, Methylnicotinat, Triethanolaminsalicylat, Glykolsalicylat, Salicylamid, Tolnaftat, Undecylensäure, Salicylsäure, Zinkundecylenat, Thiabendazol, Ivermectin, Idoxuridin, Acylcovir und Interferon bestehenden Verbindungsgruppe ausgewählt ist.

10. Zusammensetzung nach Anspruch 8, worin das antibakterielle Mittel Norfloxacin, Gentamycin oder Chloramphenicol ist, das Anästheticum Lidocain oder Benzocain ist und das entzündungshemmende Mittel Dexamethason, Indomethacin oder Sulindac ist.

11. Wässrige pharmazeutische Zusammensetzung nach Anspruch 1 zur Verabreichung in eine Körperhöhle zur Behandlung eines Zustands, der die pharmakologische Behandlung erfordert, welche
a. 10 bis 50 Gew.-% eines Polymers der Formel

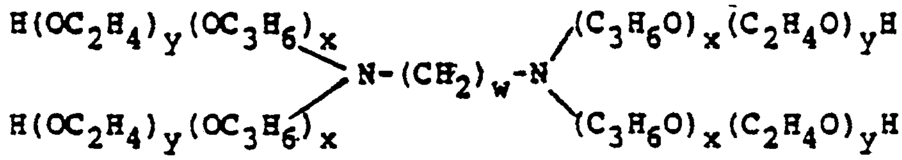

worin w eine ganze Zahl von 2 bis 6 ist, die ungefähr 40 bis 80 % Poly(oxyethylen) und ungefähr 20 bis 60 % Poly(oxypropylen) enthält und ein Molekulargewicht von 7 000 bis 50 000 hat, und worin x und y beliebige ganze Zahlen innerhalb der vorstehenden Beschränkungen sind,

b. eine pharmakologisch wirksame Menge eines aus der aus antibakteriellen Substanzen, Antihistaminica und Dekongestantien, entzündungshemmenden Mitteln, Antiparasitenmitteln, Antivirusmitteln, lokalen Anästhetica, Antipilz-, Amebecidal- oder Trichomonocidalmitteln, Analgetica, Antiarthritica, Antiasthmatica, Antikoagulantien, Antikonvulgantien, Antidepressiva, Antidiabetica, Antineoplastica, Antipsychotica, Antihypertensica und Muskelrelaxantien sowie Antiprotozoalmitteln bestehenden Gruppe ausgewählten Medikaments und

c. Salzsäure oder Natriumhydroxid in einer ausreichenden Menge, um die Flüssig-Gel-Übergangstemperatur auf über Raumtemperatur einzustellen, wobei der pH-Wert der Zusammensetzung im Bereich von 2 bis 9 liegt, umfaßt.

**12.** Zusammensetzung nach Anspruch 11, worin das Medikament aus der aus Cefoxitin, n-Formamidoylthienamycin, Tetracyclinen, Chloramphenicol, Neomycin, Gramicidin, Bacitracin, Sulfonamiden, Gentamycin, Kanamycin, Amikacin, Sisomicin, Tobramycin, Nalidixinsäuren, Norfloxacin, einer antimikrobiellen Kombination von Fludalanin/Pentizidon, Nitrofurazonen, Pyrilamin, Chlolpheniramin, Tetrahydrazolin, Antazolin, Cortison, Hydrocortison, Beta-Methason, Dexamethason, Fluocortolon, Prednisolon, Triamcinolon, Indomethacin, Sulindac, Ivermectin, Acyclovir, Interferon, Benzocain, Lidocain, Procain, Polyoxyethylennonylphenol, Alkylarylsulfonat, Oxychinolinsulfat, Miconazolnitrat, Sulfanilamid, Candicidin, Sulfisoxazol, Nystatin, Clotrimazol, Metronidazol, Chloramphenicol, Chlorochin, Trimethoprim, Sulfamethoxazol, Aspirin, Acetaminophen, Diflunisal, Lidocain, Procain, Benzocain, Xylocain, Phenylbutazon, Indomethacin, Sulindac, Dexamethason, Ibuprofen, Allopurinol, Oxyphenbutazonprobenecid, Theophyllin, Ephedrin, Beclomethasondipropionat, Epinephrin, Sulfamethoxazol, Trimethoprim, Nitrofurantoin, Norfloxicin, Heparin, Bishydroxycumarin, Warfarin, Diphenylhydantoin, Diazepam, Amitriptylin, Chlordiazepoxid, Perphenazin, Protriptylin, Imipramin, Doxepin, Insulin, Tolbutamid, Tolazamid, Acetohexamid, Chlorpropamid, Adriamycin, Fluoruracil, Methotrexat, Asparaginase, Prochlorperazin, Lithiumcarbonat, Lithiumcitrat, Thioridazin, Molindon, Fluphenazin, Trifluoperazin, Perphenazin, Amitriptylin, Triflupromazin, Spironolacton, Methyldopa, Hydralazin, Clonidin, Chlorthiazid, Deserpidin, Timolol, Propranolol, Metoprolol, Prazosinhydrochlorid, Reserpin, Melphalan, Danbrolen, Cyclobenzaprin, Methocarbamol, Diazepam, Chloramphenicol, Chlorochin, Trimethoprim und Sulfamethoxazol bestehenden Verbindungsgruppe ausgewählt ist.

**13.** Zusammensetzung nach Anspruch 11, worin das Antihypertensivum Timolol, Methyldopa oder Hydrochlorthiazid ist, das antibakterielle Mittel Norfloxacin, Chloramphenicol oder Gentamycin ist, das entzündungshemmende Mittel Dexamethason, Indomethacin oder Sulindac ist und das Anästheticum Lidocain oder Benzocain ist.

**14.** Zusammensetzung nach Anspruch 1, worin das Polymer eines ist, worin w 2 ist.

**15.** Zusammensetzung nach Anspruch 1, worin das Polymer Tetronic$^R$ 1307 ist.

**16.** Zusammensetzung nach Anspruch 1, worin die Gel-Sol-Übergangstemperatur der Zusammensetzung bei Raumtemperatur oder tiefer liegt und die Zusammensetzung bei dieser Temperatur flüssig ist.

**17.** Zusammensetzung nach Anspruch 1, welche weiterhin wenigstens ein pharmazeutisch annehmbares Pufferungsmittel, Salz und Konservierungsmittel enthält.

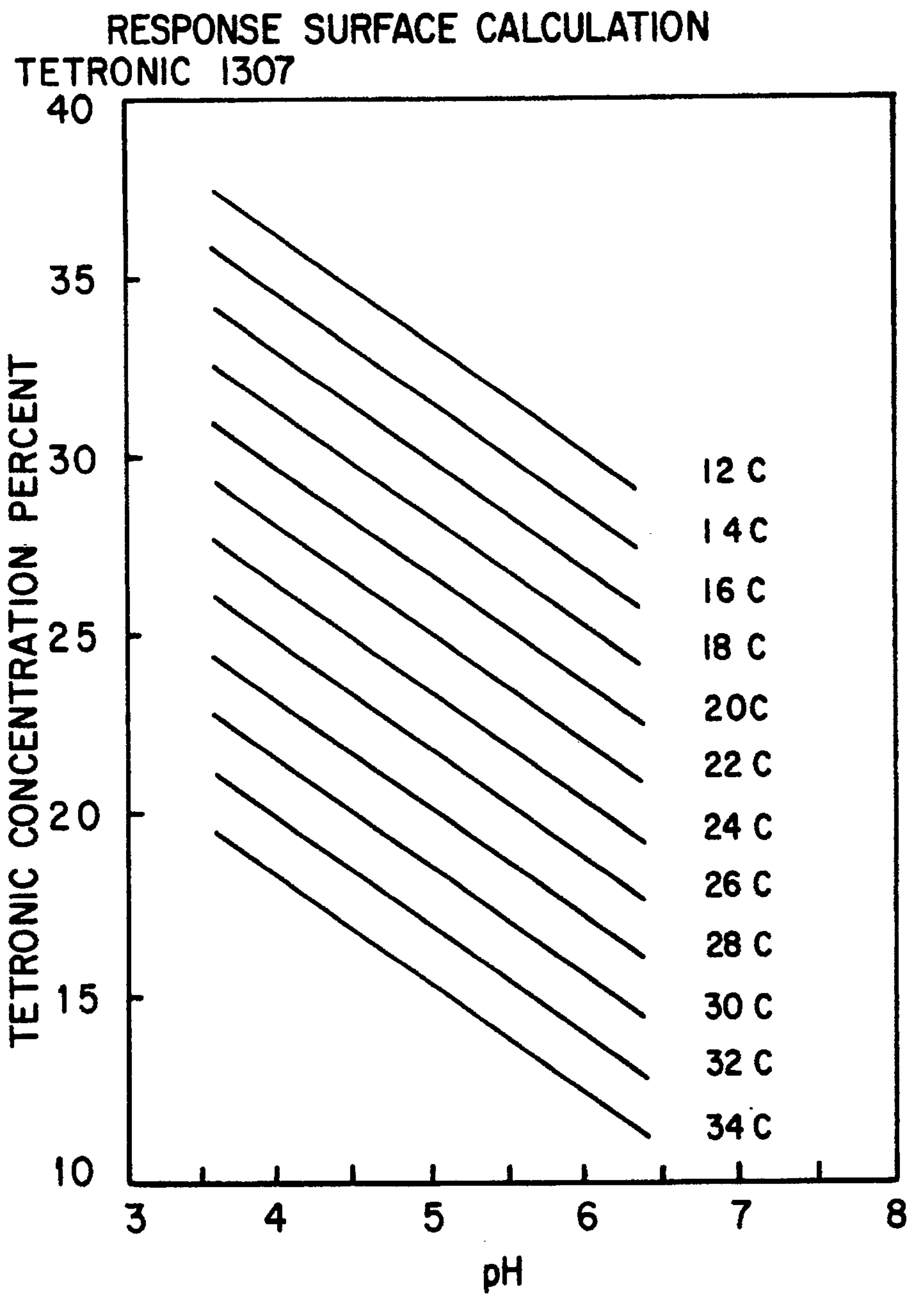
RESPONSE SURFACE CALCULATION
TETRONIC 1307
TETRONIC CONCENTRATION PERCENT
40
35
30
25
20
15
10
3
4
5
6
7
8
pH
12 C
14 C
16 C
18 C
20C
22 C
24 C
26 C
28 C
30 C
32 C
34 C

FIG. 1

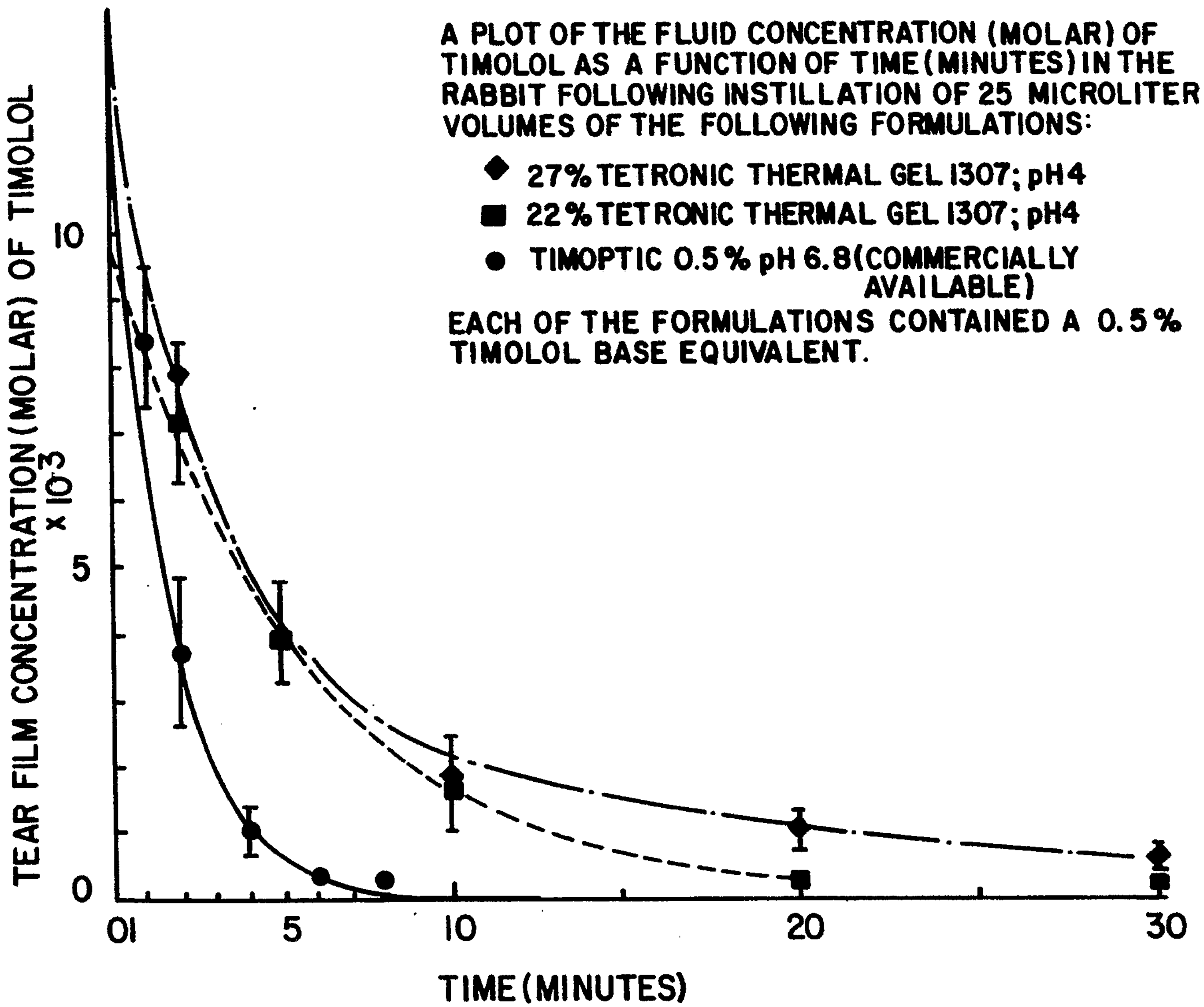
A PLOT OF THE FLUID CONCENTRATION (MOLAR) OF TIMOLOL AS A FUNCTION OF TIME (MINUTES) IN THE RABBIT FOLLOWING INSTILLATION OF 25 MICROLITER VOLUMES OF THE FOLLOWING FORMULATIONS:
◆ 27% TETRONIC THERMAL GEL 1307; pH4
■ 22% TETRONIC THERMAL GEL 1307; pH4
● TIMOPTIC 0.5% pH 6.8 (COMMERCIALLY AVAILABLE)
EACH OF THE FORMULATIONS CONTAINED A 0.5% TIMOLOL BASE EQUIVALENT.
TEAR FILM CONCENTRATION (MOLAR) OF TIMOLOL
x 10-3
10
5
0
01
5
10
20
30
TIME (MINUTES)

FIG.2